# EUROPEAN PATENT SPECIFICATION

(11) **EP 2 890 420 B1**
(45) Date of publication and mention of the grant of the patent: **29.08.2018**
(21) Application number: 13832809.1
(22) Date of filing: 30.08.2013
(51) Int. Cl.: A61M 1/12, A61M 1/10

(54) **INSTABILITY DETECTION ALGORITHM FOR AN IMPLANTABLE BLOOD PUMP**
INSTABILITÄTSERKENNUNGSALGORITHMUS FÜR EINE IMPLANTIERBARE BLUTPUMPE
ALGORITHME DE DÉTECTION D'INSTABILITÉ DESTINÉ À UNE POMPE À SANG IMPLANTABLE

(30) Priority: 31.08.2012 US 201261695476 P
(43) Date of publication of application: 08.07.2015
(73) Proprietor: TC1 LLC, St. Paul, MN 55117 (US)
(72) Inventor: SIEBENHAAR, Andre, CH-5073 Gipf-Oberfrick (CH)
(74) Representative: Peterreins Schley
(86) International application number: PCT/US2013/057548
(87) International publication number: WO 2014/036416

(56) References cited:
- WO-A1-2007/105842
- WO-A2-99/53974
- US-A- 6 071 093
- US-A1- 2001 031 210
- US-A1- 2005 014 991
- US-A1- 2005 135 948
- US-A1- 2012 046 514
- US-A1- 2012 126 795
- US-A1- 2012 156 071
- US-A1- 2012 207 630
- US-B1- 6 866 625

## Description

### CROSS-REFERENCE TO RELATED APPLICATIONS

This application claims priority to and the full benefit of U.S. Provisional Application Serial No. 61/695,476, filed August 31, 2012.

The description relates to United States Published Application No. 2012/0046514, filed August 18, 2011, and titled "Implantable Blood Pump,".

### FIELD

This description relates to motor control, and in various respects an instability detection algorithm, for an implantable blood pump.

### BACKGROUND

Ventricular assist devices, known as VADs, are implantable blood pumps used for both short-term and long-term applications where a patient's heart is incapable of providing adequate circulation. For example, a patient suffering from heart failure may use a VAD while awaiting a heart transplant. In another example, a patient may use a VAD while recovering from heart surgery. Thus, a VAD can supplement a weak heart or can effectively replace the natural heart's function. VADs can be implanted in the patient's body and powered by an electrical power source inside or outside the patient's body.

Some implantable blood pumps (see e.g. US 2012/207630) employ rotor designs where the rotor needs to be released from magnetic attraction before the rotor can be activated by a motor. For such starting algorithms of a rotor in an implantable blood pump, significant currents are required that may require heavyweight power supplies.

### SUMMARY

The present disclosure describes one or more general aspects, implementations or embodiments involving devices, systems and methods for an instability detection algorithm for an implantable blood pump. The invention is defined by independent claim 1.

One or more of the following aspects of this disclosure can be embodied as methods that include the corresponding actions. One or more of the following aspects of this disclosure can be implemented in a device including a processor, a processor-readable medium coupled to the processor having instructions stored thereon which, when executed by the processor, cause the processor to perform operations according to the one or more of the following aspects. One or more of the following aspects of this disclosure can be implemented on a computer-readable medium having instructions stored thereon that, when executed by a processor, cause the processor to perform operations according to the one or more of the following aspects. Where in the following the word invention is used and/or features are presented as optional this should be interpreted in such a way that only protection is sought for the invention as claimed.

In aspect 1, a blood pump system is comprising: a housing; a rotary motor comprising a stator and a rotor, the rotor having permanent magnetic poles for magnetic levitation of the rotor; a controller configured to perform the following operations: determining a target position of the rotor; calculating a positional displacement of the target position from a predefined origin of a coordinate system of the housing; calculating, during a rotation of the rotor, geometric deviations of a current position of the rotor from the target position.

Aspect 2 according to aspect 1, wherein the controller is further configured to perform the following operations: generating translatory instructions to initiate a translational movement of the rotor to the target position, wherein, when the rotor is located within a predetermined volume around the target position, sending a start command to the rotary motor to start rotating the rotor.

Aspect 3 according to aspect 1 or 2, wherein the controller is further configured to perform the following operations: outputting the positional displacement and a figure of merit for a stability of the rotor based on the geometric deviations.

Aspect 4 according to any one of aspects 1 to 3, wherein the target position is a position within the housing where a power consumption for the magnetic levitation is calculated to be below a predefined power threshold.

Aspect 5 according to any one of aspects 1 to 4, wherein the target position is a position within the housing at which a DC component of a levitation current of the rotor is calculated to be below a predefined DC current threshold.

Aspect 6 according to aspect 3, wherein the figure of merit is a moving average of absolute magnitudes of the geometric displacements, wherein the geometric deviations are calculated with a sampling rate of about 20 kilohertz.

Aspect 7 according to any one of aspects 1 to 6, wherein the housing is an implantable blood pump housing, wherein the rotary motor and the controller are positioned within the implantable blood pump housing.

Aspect 8 according to any one of aspects 1 to 7, wherein the positional displacements and the geometric deviations are calculated based on an output voltage of a Hall sensor that is included in the housing.

In aspect 9, a method implemented in an implantable blood pump, the method is comprising: determining a target position of a rotor of the pump, the rotor having permanent magnetic poles for magnetic levitation of the rotor; calculating a positional displacement of the target position from a predefined origin of a coordinate system of a housing of the pump; calculating, during a rotation of the rotor, geometric deviations of a current position of the rotor from the target position.

Aspect 10 according to aspect 9, further comprising: generating translatory instructions to initiate a translational movement of the rotor to the target position, wherein, when the rotor is located within a predetermined volume around the target position, sending a start command to the rotary motor to start rotating the rotor.

Aspect 11 according to any one of aspects 9 to 10, further comprising: outputting the positional displacement and a figure of merit for a stability of the rotor based on the geometric deviations.

Aspect 12 according to any one of aspects 9 to 11, wherein the target position is a position within the housing where a power consumption for the magnetic levitation is calculated to be below a predefined power threshold.

Aspect 13 according to any one of aspects 9 to 12, wherein the target position is a position within the housing at which a DC component of a levitation current of the rotor is calculated to be below a predefined DC current threshold.

Aspect 14 according to aspect 11, wherein the figure of merit is a moving average of absolute magnitudes of the geometric displacements, wherein the geometric deviations are calculated with a sampling rate of about 20 kilohertz.

Aspect 15 according to any one of aspects 9 to 14, wherein the housing is an implantable blood pump housing, wherein the rotary motor and the rotor are positioned within the implantable blood pump housing.

Aspect 16 according to any one of aspects 9 to 15, wherein the positional displacements and the geometric deviations are calculated based on an output voltage of a Hall sensor that is included in the housing.

In aspect 17 a computer-readable medium is having computer-executable instructions stored thereon that, when executed by a processor, cause the processor to perform operations, comprising: determining a target position of a rotor of an implantable blood pump, the rotor having permanent magnetic poles for magnetic levitation of the rotor; calculating a positional displacement of the target position from a predefined origin of a coordinate system of a housing of the pump; calculating, during a rotation of the rotor, geometric deviations of a current position of the rotor from the target position.

In aspect 18, that is combinable with any one of aspects 2 to 8, a blood pump system is comprising: a housing; a rotary motor comprising a stator and a rotor, the rotor having permanent magnetic poles for magnetic levitation of the rotor; a controller configured to perform the following operations: determining a target position of the rotor; calculating a positional displacement of the target position from a predefined origin of a coordinate system of the housing.

In aspect 19, that is combinable with any one of aspects 2 to 8, a blood pump system is comprising: a housing; a rotary motor comprising a stator and a rotor, the rotor having permanent magnetic poles for magnetic levitation of the rotor; a controller configured to perform the following operations: calculating, during a rotation of the rotor, geometric deviations of a current position of the rotor from a predefined target position.

In aspect 20, aspect 17 is implemented in the controller of any one of aspects 1 to 8 and aspects 18 to 19.

The following general aspects may be combinable with any one of the aspects 1 to 20.

In one general aspect, an implantable blood pump includes a housing and a blood flow conduit. Within the housing, the blood pump includes a stator located about the blood flow conduit and a magnetically-levitated rotor.

In another general aspect, an implantable blood pump includes a housing defining an inlet opening and an outlet opening. Within the housing, a dividing wall defines a blood flow conduit extending between the inlet opening and the outlet opening of the housing. The blood pump has a rotary motor that includes a stator and a rotor. The stator is disposed within the housing circumferentially about the dividing wall such that the inner blood flow conduit extends through the stator.

In another general aspect, an implantable blood pump includes a puck-shaped housing having a first face defining an inlet opening, a peripheral sidewall, and a second face opposing the first face. The blood pump has an internal dividing wall defining an inner blood flow conduit extending between the inlet opening and an outlet opening of the housing. The puck-shaped housing has a thickness from the first face to the second face that is less than a width of the housing between opposing portions of the peripheral sidewall. The blood pump also has a motor having a stator and a rotor. The stator is disposed in the housing circumferentially about the blood flow conduit and includes magnetic levitation components operable to control an axial position and a radial position of the rotor. The rotor is disposed in the inner blood flow conduit and includes an impeller operable to pump blood from the inlet opening to the outlet opening through at least a portion of the magnetic levitation components of the stator.

Implementations of the above aspects may include one or more of the following features. For example, the stator is disposed circumferentially about at least a part of the rotor and is positioned relative to the rotor such that in use blood flows within the blood flow conduit through the stator before reaching the rotor. The rotor has permanent magnetic poles for magnetic levitation of the rotor. A passive magnetic control system is configured to control an axial position of the rotor relative to the stator, and an active electromagnetic control system is configured to radially center the rotor within the inner blood flow conduit. An electromagnetic control system controls at least one of a radial position and an axial position of the rotor relative to the stator, and the electromagnetic control system has control electronics located within the housing about the dividing wall.

The control electronics are located between the inlet opening and the stator. The control electronics can be configured to control the active magnetic control system. The rotor has only one magnetic moment. The stator includes a first coil for driving the rotor and a second coil for controlling a radial position of the rotor, and the first coil and the second coil are wound around a first pole piece of the stator. The housing has a first face that defines the inlet opening, a second face opposing the first face, and a peripheral wall extending from the first face to the second face. The housing includes a rounded transition from the second face to the peripheral wall. The housing defines a volute located such that in use blood flows within the blood flow conduit through the stator before reaching the volute. The volute can be located between the stator and the second face. The housing can also include a cap that includes the second face, defines at least part of the volute, and defines at least part of the outlet. The cap is engaged with the peripheral wall of the housing. The housing also includes an inlet cannula extending from the first face and in fluid communication with the inlet opening. The inlet cannula can be inserted into the patient's heart. The outlet opening is defined in the second face and/or the peripheral wall. A thickness of the housing between the first face and the second face is less than a width of the housing.

In another general aspect, a method includes inserting a puck-shaped blood pump housing into a patient's body. The blood pump is inserted such that an opening defined in a first flat face of the housing that is proximate to a stator of the blood pump faces the patient's heart. Additionally, the blood pump is inserted such that a second rounded face of the housing that is proximate to an impeller of the blood pump faces away from the patient's heart. The first face is disposed against a portion of the patient's heart such that the second face of the housing faces away from the heart of the patient. In some implementations, the method includes inserting an inlet cannula of the housing into the patient's heart.

In another general aspect, making a blood pump includes assembling a motor stator and control electronics in a puck-shaped housing circumferentially about an internal dividing wall. The internal dividing wall defines an inner blood flow conduit that extends from an inlet opening to an outlet opening of the housing. The stator is assembled in the housing such that the inner blood flow conduit extends through the motor stator. Disposed within the inner blood flow conduit is a magnetically-levitated rotor. The rotor is surrounded by the stator such that impeller blades carried by the rotor are downstream of the stator from the inlet opening. In use, the impeller pumps blood from the inlet opening to the outlet opening through the stator.

Implementations may include one or more of the following features, For example, the rotor has only one magnetic moment. The stator includes at least one first coil for driving the rotor and at least one second coil for controlling a radial position of the rotor, the at least one first coil and the at least one second coil being wound around a first pole piece of the stator. The housing includes a first face that defines the inlet opening, and further comprising engaging an end cap with a peripheral wall of the housing, the end cap including a second face, defining at least part of a volute, and defining at least part of the outlet opening. The housing includes a rounded transition from the second face to the peripheral wall. The housing further includes an inlet cannula extending from the first face and in fluid communication with the inlet opening. A thickness of the housing between the first face and the second face is less than a width of the housing.

In another general aspect, a method of pumping blood includes magnetically rotating a centrifugal pump impeller of a blood pump device to draw blood from a patient's heart through an inlet opening of a housing of the blood pump device into an inner blood flow conduit within a stator in the housing, through the inner blood flow conduit, and through an outlet opening of the housing. The method includes selectively controlling a radial position of the impeller within the inner blood flow conduit.

The details of one or more of these and other aspects, implementations or embodiments are set forth in the accompanying drawings and the description below. Other features, aims and advantages will be apparent from the description and drawings, and from the claims.

### DESCRIPTION OF THE DRAWINGS

FIG. 1 is an illustration of a blood pump in a use position implanted in a patient's body.
FIG. 2 is a cross-sectional view of the blood pump of FIG. 1.
FIG. 3 is a partial cut-away perspective view of a stator of a blood pump.
FIG. 4 is a bottom perspective view of a blood pump.
FIG. 5 is a top perspective view of the blood pump of FIG. 4.
FIG. 6 describes a current consumption of a standard start-up that is successful in this example.
FIG. 7 describes an exemplary positional alignment of a rotor with respect to a housing of the pump.
FIG. 8 describes an exemplary geometrical alignment of a rotor with respect to a housing of the pump.
FIG. 9 describes a process flow of an exemplary instability detection algorithm.

Reference numbers and designations in the various drawings indicate exemplary aspects, implementations or embodiments of particular features of the present disclosure.

### DETAILED DESCRIPTION

This description relates to an improved instability detection algorithm for an implantable blood pump. Specifically, tools and computer-implemented methods for an instability detection algorithm are integrated at an implantable blood pump. The instability detection algorithm monitors a performance of a rotor of the implantable blood pump.

The subject matter described in this disclosure can be implemented in particular aspects or embodiments so as to realize one or more of the following advantages.

First, positional measures of a rotor in an implantable blood pump may be monitored during operation of the pump. For example, rotor displacements or noise of the rotor position during operation of the pump may be sensed and provided to a control unit that may be external to the body of a patient carrying the pump. A stable rotation of the rotor may reduce current consumption of the pump, e.g. by reducing the number of required restarts of the rotor and consequently the number of required take-offs. This may circumvent high current consumption and high peak currents during the operation of the rotor.

Second, a target position of a rotor of the implantable blood pump may be computed, wherein the target position is a position within a flow conduit of the pump where power consumption for rotor levitation may be reduced. For example, the target position may be a position within the flow conduit at which a DC component of a levitation current of the rotor is computed to be minimal.

Third, a gap for a fluid (e.g., blood) to pass the rotor in the fluid conduit may be computed from the target position. For example, the gap may be between an inner wall of the pump and a magnet of the rotor. For example, it may be derived from the gap if sufficient blood can pass through the gap to fulfill a certain predefined blood flow through the pump. For instance, hydrodynamic measures may be computed from the gap and/or the target position. This provides information for adjusting one or more functional parameters of the pump to optimize operation of the pump.

Fourth, the computing of the target position may provide information about a magnetic field distributed within the pump. For example, a strength and/or field lines of a magnetic field inside the pump may be computed from the target position. This provides data for adjusting one or more functional parameters of the pump to optimize operation of the pump.

Other advantages of this disclosure will be apparent to those skilled in the art.

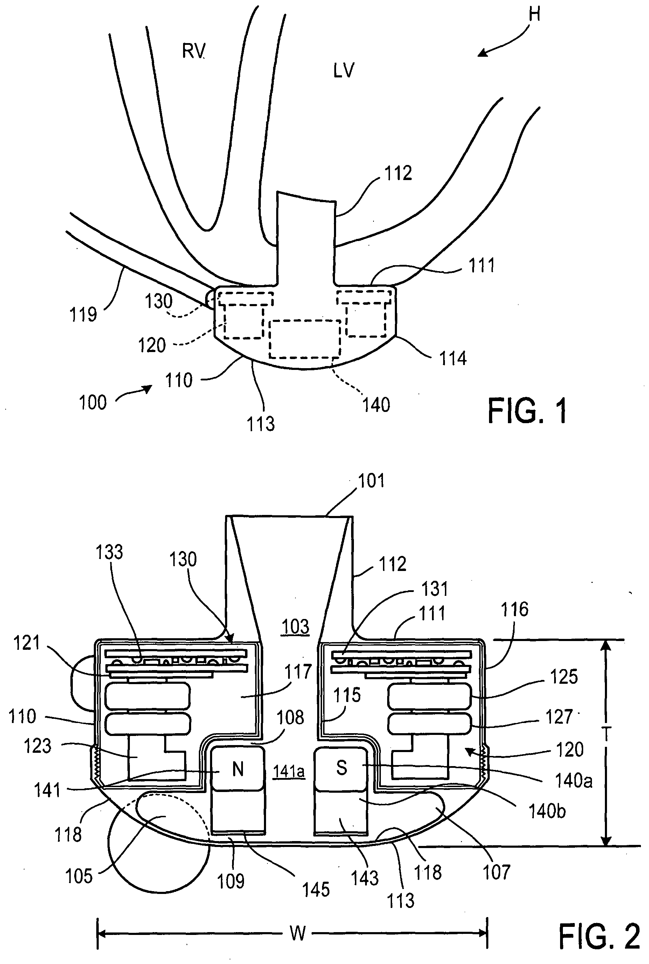

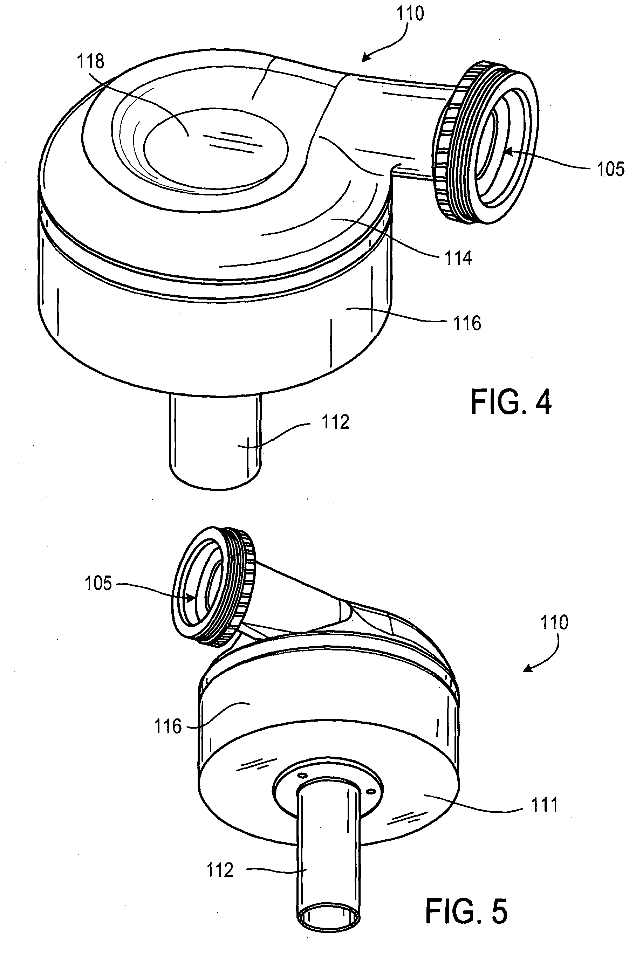

With reference to FIGS. 1 to 5, a left ventricular assist blood pump 100 having a puck-shaped housing 110 is implanted in a patient's body with a first face 111 of the housing 110 positioned against the patient's heart H and a second face 113 of the housing 110 facing away from the heart H. The first face 111 of the housing 110 includes an inlet cannula 112 extending into the left ventricle LV of the heart H. The second face 113 of the housing 110 has a chamfered edge 114 to avoid irritating other tissue that may come into contact with the blood pump 100, such as the patient's diaphragm. To construct the illustrated shape of the puck-shaped housing 110 in a compact form, a stator 120 and electronics 130 of the pump 100 are positioned on the inflow side of the housing toward first face 111, and a rotor 140 of the pump 100 is positioned along the second face 113. This positioning of the stator 120, electronics 130, and rotor 140 permits the edge 114 to be chamfered along the contour of the rotor 140, as illustrated in at least Figs. 2, 4, and 5, for example.

Referring to FIG. 2, the blood pump 100 includes a dividing wall 115 within the housing 110 defining a blood flow conduit 103. The blood flow conduit 103 extends from an inlet opening 101 of the inlet cannula 112 through the stator 120 to an outlet opening 105 defined by the housing 110. The rotor 140 is positioned within the blood flow conduit 103. The stator 120 is disposed circumferentially about a first portion 140a of the rotor 140, for example about a permanent magnet 141. The stator 120 is also positioned relative to the rotor 140 such that, in use, blood flows within the blood flow conduit 103 through the stator 120 before reaching the rotor 140. The permanent magnet 141 has a permanent magnetic north pole N and a permanent magnetic south pole S for combined active and passive magnetic levitation of the rotor 140 and for rotation of the rotor 140. The rotor 140 also has a second portion 140b that includes impeller blades 143. The impeller blades 143 are located within a volute 107 of the blood flow conduit such that the impeller blades 143 are located proximate to the second face 113 of the housing 110.

The puck-shaped housing 110 further includes a peripheral wall 116 that extends between the first face 111 and a removable cap 118. As illustrated, the peripheral wall 116 is formed as a hollow circular cylinder having a width W between opposing portions of the peripheral wall 116. The housing 110 also has a thickness T between the first face 111 and the second face 113 that is less than the width W. The thickness T is from about 0.5 inches to about 1.5 inches, and the width W is from about 1 inch to about 4 inches. For example, the width W can be approximately 2 inches, and the thickness T can be approximately 1 inch.

The peripheral wall 116 encloses an internal compartment 117 that surrounds the dividing wall 115 and the blood flow conduit 103, with the stator 120 and the electronics 130 disposed in the internal compartment 117 about the dividing wall 115. The removable cap 118 includes the second face 113, the chamfered edge 114, and defines the outlet opening 105. The cap 118 can be threadedly engaged with the peripheral wall 116 to seal the cap 118 in engagement with the peripheral wall 116. The cap 118 includes an inner surface 118a of the cap 118 that defines the volute 107 that is in fluid communication with the outlet opening 105.

Within the internal compartment 117, the electronics 130 are positioned adjacent to the first face 111 and the stator 120 is positioned adjacent to the electronics 130 on an opposite side of the electronics 130 from the first face 111. The electronics 130 include circuit boards 131 and various components carried on the circuit boards 131 to control the operation of the pump 100 by controlling the electrical supply to the stator 120. The housing 110 is configured to receive the circuit boards 131 within the internal compartment 117 generally parallel to the first face 111 for efficient use of the space within the internal compartment 117. The circuit boards also extend radially-inward towards the dividing wall 115 and radially-outward towards the peripheral wall 116. For example, the internal compartment 117 is generally sized no larger than necessary to accommodate the circuit boards 131, and space for heat dissipation, material expansion, potting materials, and/or other elements used in installing the circuit boards 131. Thus, the external shape of the housing 110 proximate the first face 111 generally fits the shape of the circuits boards 131 closely to provide external dimensions that are not much greater than the dimensions of the circuit boards 131.

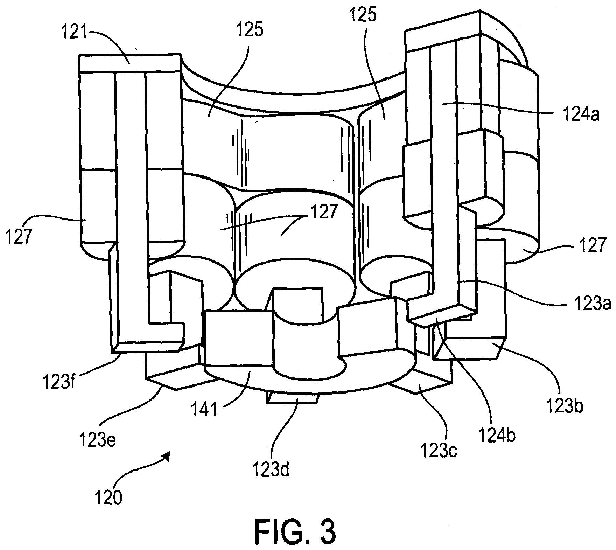

With continued reference to FIG. 2 and with reference to FIG. 3, the stator 120 includes a back iron 121 and pole pieces 123a-123f arranged at intervals around the dividing wall 115. The back iron 121 extends around the dividing wall 115 and is formed as a generally flat disc of a ferromagnetic material, such as steel, in order to conduct magnetic flux. The back iron 121 is arranged beside the control electronics 130 and provides a base for the pole pieces 123a-123f.

Each of the pole piece 123a-123f is L-shaped and has a drive coil 125 for generating an electromagnetic field to rotate the rotor 140. For example, the pole piece 123a has a first leg 124a that contacts the back iron 121 and extends from the back iron 121 towards the second face 113. The pole piece 123a may also have a second leg 124b that extends from the first leg 124a through an opening of a circuit board 131 towards the dividing wall 115 proximate the location of the permanent magnet 141 of the rotor 140. In an aspect, each of the second legs 124b of the pole pieces 123a-123f is sticking through an opening of the circuit board 131. In an aspect, each of the first legs 124a of the pole pieces 123a-123f is sticking through an opening of the circuit board 131. In an aspect, the openings of the circuit board are enclosing the first legs 124a of the pole pieces 123a-123f.

In a general aspect, the implantable blood pump 100 may include a Hall sensor that may provide an output voltage, which is directly proportional to a strength of a magnetic field that is located in between at least one of the pole pieces 123a-123f and the permanent magnet 141, and the output voltage may provide feedback to the control electronics 130 of the pump 100 to determine if the rotor 140 and/or the permanent magnet 141 is not at its intended position for the operation of the pump 100. For example, a position of the rotor 140 and/or the permanent magnet 141 may be adjusted, e.g. the rotor 140 or the permanent magnet 141 may be pushed or pulled towards a center of the blood flow conduit 103 or towards a center of the stator 120.

Each of the pole pieces 123a-123f also has a levitation coil 127 for generating an electromagnetic field to control the radial position of the rotor 140. Each of the drive coils 125 and the levitation coils 127 includes multiple windings of a conductor around the pole pieces 123a-123f. Particularly, each of the drive coils 125 is wound around two adjacent ones of the pole pieces 123, such as pole pieces 123d and 123e, and each levitation coil 127 is wound around a single pole piece. The drive coils 125 and the levitation coils 127 are wound around the first legs of the pole pieces 123, and magnetic flux generated by passing electrical current though the coils 125 and 127 during use is conducted through the first legs and the second legs of the pole pieces 123 and the back iron 121. The drive coils 125 and the levitation coils 127 of the stator 120 are arranged in opposing pairs and are controlled to drive the rotor and to radially levitate the rotor 140 by generating electromagnetic fields that interact with the permanent magnetic poles S and N of the permanent magnet 141. Because the stator 120 includes both the drive coils 125 and the levitation coils 127, only a single stator is needed to levitate the rotor 140 using only passive and active magnetic forces. The permanent magnet 141 in this configuration has only one magnetic moment and is formed from a monolithic permanent magnetic body 141. For example, the stator 120 can be controlled as discussed in U.S. Patent No. 6,351,048. The control electronics 130 and the stator 120 receive electrical power from a remote power supply via a cable 119 (FIG. 1). Further related patents, namely U.S. Patent No. 5,708,346, U.S. Patent No. 6,053,705, U.S. Patent No. 6,100,618, U.S. Patent No. 6,879,074, U.S. Patent No. 7,112,903, U.S. Patent No. 6,278,251, U.S. Patent No. 6,278,251, U.S. Patent No. 6,351,048, U.S. Patent No. 6,249,067, U.S. Patent No. 6,222,290, U.S. Patent No. 6,355,998 and U.S. Patent No. 6,634,224.

The rotor 140 is arranged within the housing 110 such that its permanent magnet 141 is located upstream of impeller blades in a location closer to the inlet opening 101. The permanent magnet 141 is received within the blood flow conduit 103 proximate the second legs 124b of the pole pieces 123 to provide the passive axial centering force though interaction of the permanent magnet 141 and ferromagnetic material of the pole pieces 123. The permanent magnet 141 of the rotor 140 and the dividing wall 115 form a gap 108 between the permanent magnet 141 and the dividing wall 115 when the rotor 140 is centered within the dividing wall 115. The gap 108 may be from about 0.2 millimeters to about 2 millimeters. For example, the gap 108 is approximately 1 millimeter. The north permanent magnetic pole N and the south permanent magnetic pole S of the permanent magnet 141 provide a permanent magnetic attractive force between the rotor 140 and the stator 120 that acts as a passive axial centering force that tends to maintain the rotor 140 generally centered within the stator 120 and tends to resist the rotor 140 from moving towards the first face 111 or towards the second face 113. When the gap 108 is smaller, the magnetic attractive force between the permanent magnet 141 and the stator 120 is greater, and the gap 108 is sized to allow the permanent magnet 141 to provide the passive magnetic axial centering force having a magnitude that is adequate to limit the rotor 140 from contacting the dividing wall 115 or the inner surface 118a of the cap 118. The rotor 140 also includes a shroud 145 that covers the ends of the impeller blades 143 facing the second face 113 that assists in directing blood flow into the volute 107. The shroud 145 and the inner surface 118a of the cap 118 form a gap 109 between the shroud 145 and the inner surface 118a when the rotor 140 is levitated by the stator 120. The gap 109 is from about 0.2 millimeters to about 2 millimeters. For example, the gap 109 is approximately 1 millimeter.

As blood flows through the blood flow conduit 103, blood flows through a central aperture 141 a formed through the permanent magnet 141. Blood also flows through the gap 108 between the rotor 140 and the dividing wall 115 and through the gap 109 between the shroud 145 and the inner surface 108a of the cap 118. The gaps 108 and 109 are large enough to allow adequate blood flow to limit clot formation that may occur if the blood is allowed to become stagnant. The gaps 108 and 109 are also large enough to limit pressure forces on the blood cells such that the blood is not damaged when flowing through the pump 100. As a result of the size of the gaps 108 and 109 limiting pressure forces on the blood cells, the gaps 108 and 109 are too large to provide a meaningful hydrodynamic suspension effect. That is to say, the blood does not act as a bearing within the gaps 108 and 109, and the rotor is only magnetically-levitated. In various embodiments, the gaps 108 and 109 are sized and dimensioned so the blood flowing. through the gaps forms a film that provides a hydrodynamic suspension effect. In this manner, the rotor can be suspended by magnetic forces, hydrodynamic forces, or both.

Because the rotor 140 is radially suspended by active control of the levitation coils 127 as discussed above, and because the rotor 140 is axially suspended by passive interaction of the permanent magnet 141 and the stator 120, no rotor levitation components are needed proximate the second face 113. The incorporation of all the components for rotor levitation in the stator 120 (i.e., the levitation coils 127 and the pole pieces 123) allows the cap 118 to be contoured to the shape of the impeller blades 143 and the volute 107. Additionally, incorporation of all the rotor levitation components in the stator 120 eliminates the need for electrical connectors extending from the compartment 117 to the cap 118, which allows the cap to be easily installed and/or removed and eliminates potential sources of pump failure.

In use, the drive coils 125 of the stator 120 generates electromagnetic fields through the pole pieces 123 that selectively attract and repel the magnetic north pole N and the magnetic south pole S of the rotor 140 to cause the rotor 140 to rotate within stator 120. For example, the Hall sensor may sense a current position of the rotor 140 and/or the permanent magnet 141, wherein the output voltage of the Hall sensor may be used to selectively attract and repel the magnetic north pole N and the magnetic south pole S of the rotor 140 to cause the rotor 140 to rotate within stator 120. As the rotor 140 rotates, the impeller blades 143 force blood into the volute 107 such that blood is forced out of the outlet opening 105. Additionally, the rotor draws blood into pump 100 through the inlet opening 101. As blood is drawn into the blood pump by rotation of the impeller blades 143 of the rotor 140, the blood flows through the inlet opening 101 and flows through the control electronics 130 and the stator 120 toward the rotor 140. Blood flows through the aperture 141a of the permanent magnet 141 and between the impeller blades 143, the shroud 145, and the permanent magnet 141, and into the volute 107. Blood also flows around the rotor 140, through the gap 108 and through the gap 109 between the shroud 145 and the inner surface 118a of the cap 118. The blood exits the volute 107 through the outlet opening 105.

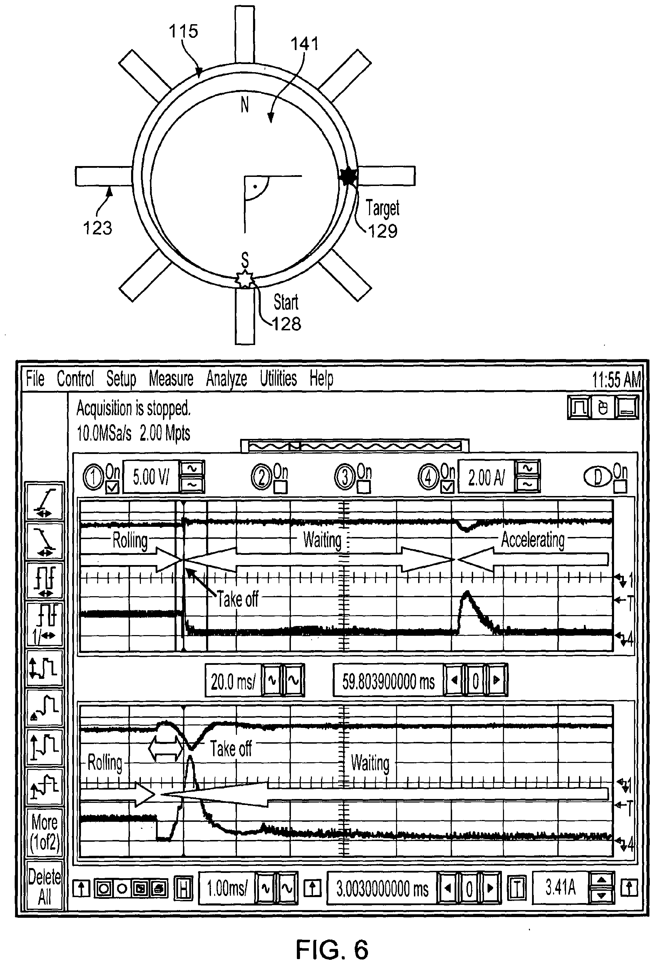

FIG. 6 describes a current consumption of a standard start-up procedure that is successful in this example. Start-up procedures may comprise a rolling phase, a take-off, a waiting phase and an accelerating phase. At the beginning of the start-up, the permanent magnet 141 is magnetically attracted by the pole pieces 123a-f and/or the coils 125, 127. The magnet 141 may contact the wall 115 or one or more pole pieces 123 of the stator 120 at a start point 135. In the rolling phase the control electronics of the implantable blood pump 100 may roll the permanent magnet 141 of the pump 100 along a wall 115 or along a stator 120 to reduce a magnetic attraction the magnet 141 experiences due to the presence of one or more of the pole pieces 123a-123f and/or one or more of the coils 125, 127. The aim of the rolling phase is to align the permanent magnet 141 of the rotor 140 in such a way that the equator (i.e., the direction where the magnetic field of the magnet 141 is vanishing) of the magnet faces or touches the contact or start position 135. The part of the magnet 141 formerly contacting the wall 115 or one or more pole pieces 123 of the stator 120 at the start position 135 is then after rolling contacting the wall 115 or one or more pole pieces 123 of the stator 120 at a position between two pole pieces. For instance, it may also be the case that to rotor 140 does not touch the wall 115 with a pole of the magnet 141 but with a part of the magnet not being one of the poles. Rolling as understood in this disclosure can include a rotation of the rotor, a sliding of the rotor along a wall of the housing, a rolling along the wall or a combination thereof.

In an aspect, if the electronics of the pump provide sufficient current, a force may be applied to the magnet 141 to remove the magnet from the wall 115 or the stator 120 during the take-off. After the take-off and the waiting phase, the rotor is accelerated to the nominal rotation speed of the operational pump 100. FIG. 6 illustrates that the peak current during the take-off may exceed the peak current during the accelerating phase and thereby illustrates that a stable rotation of the rotor 140 reduces the current consumption of the motor of the pump 100 by reducing the number of required restarts of the rotor and consequently the number of required take-offs. This may circumvent high current consumption and high peak currents during the operation of the rotor.

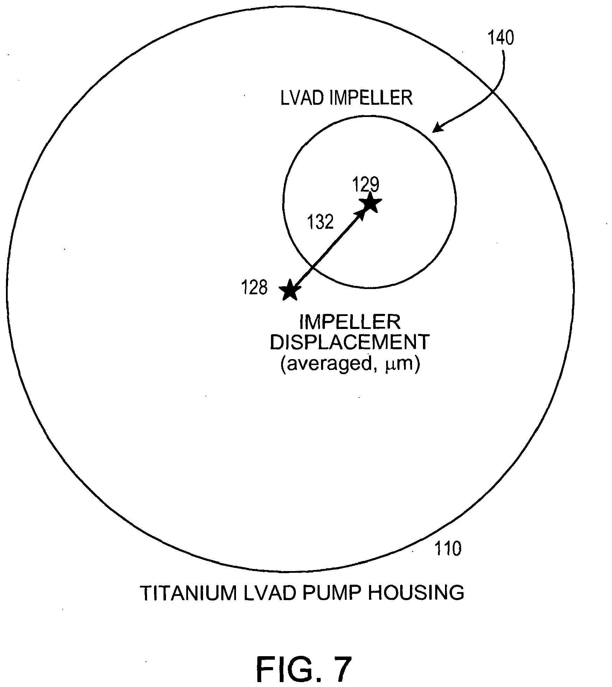

FIG. 7 describes an exemplary alignment of the rotor with respect to the housing 110 of the pump 100. The housing may be described by a coordinate system (e.g., a Cartesian coordinate system) with an origin 128. The rotor 140 may be placed at a target position 129, which is separated from the origin 128 by a displacement 132. For example, the target position 129 may be a position within the flow conduit 103 of the pump 100, wherein at the target position 129 a power consumption for the rotor levitation may be reduced. For example, the target position 129 may be a position within the flow conduit 103 at which a DC component of a current required for levitation of the rotor 140 is minimal. The target position 129 may be located at a magnetic center of the magnetic field within the stator 120, which may be displaced from the origin 128 by the displacement 132.

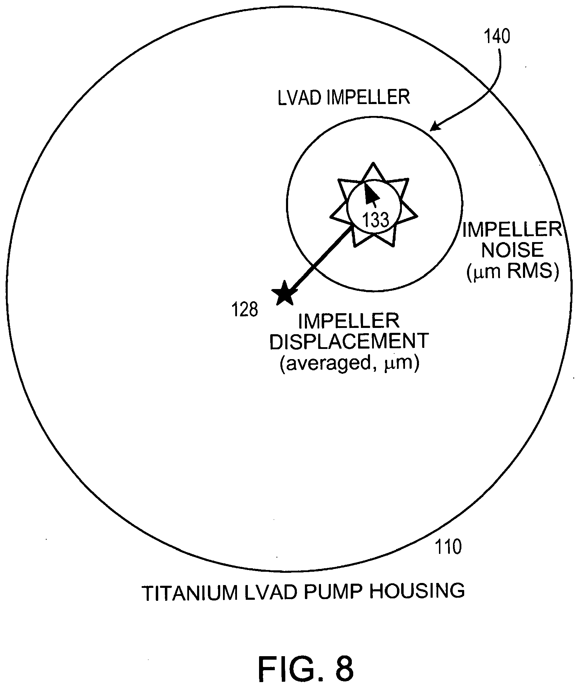

FIG. 8 continues describing the exemplary alignment of the rotor with respect to the housing 110 of the pump 100. A positional noise 133 may be computed by the instability detection algorithm. For example, positional deviations of the rotor 140 from the target position 129 may be computed during an operation of the pump 100, e.g. during an operation of the rotor. This positional noise 133 may provide a measure for the instability of the rotor during its operation. In an aspect, the algorithm may initiate a monitoring of the positional deviations of the rotor 140 from the target position 129 in two radial directions during the operation of the rotor. For example, the two radial directions may be orthogonal to each other. For example the two radial directions may lie in a plane that is perpendicular to the rotation axis of the rotor. For example the two radial directions may lie in a plane that is perpendicular to the symmetry axis of the housing 110. In an aspect, an absolute magnitude of the positional deviation of the rotor from the target position 129 may be averaged over many sampled positions of the rotor. In a general aspect, the Hall sensor described above may be used to measure or sample the positions of the rotor. For example the output voltage of the Hall sensor may provide a measure for the positional deviation of the rotor from the target position 129. For example the output voltage of the Hall sensor may provide a measure for the displacement 132 of the rotor from the origin 128. The output voltage of the Hall sensor may serve as input for the algorithm 900. In an aspect, the Hall sensor and the algorithm are implemented at the implantable blood pump. In an aspect, the algorithm is implemented at the blood pump, in an internal controller off the pump, an external controller, or a combination thereof.

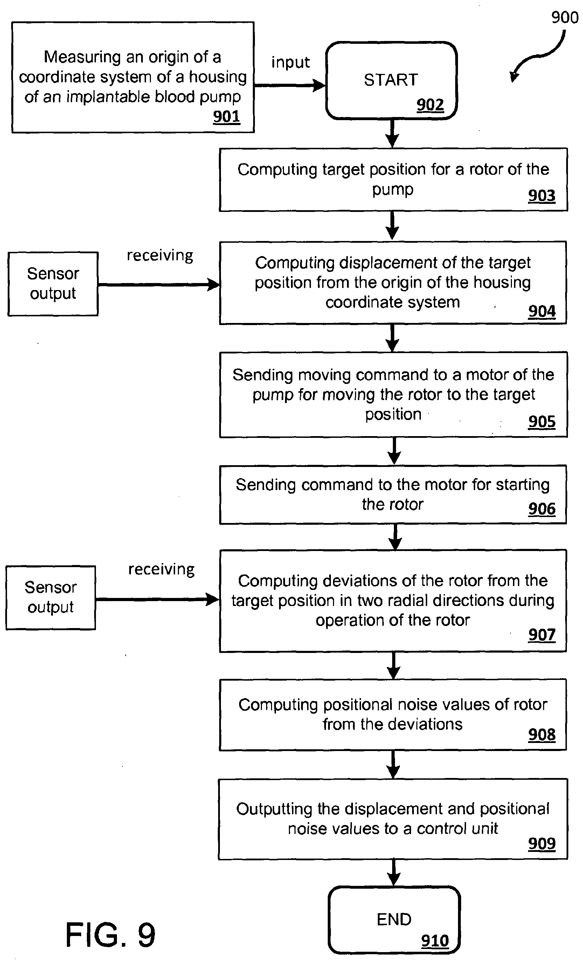

FIG. 9 describes a process flow of an exemplary instability detection algorithm according to one or more aspects described herein. At step 901, an origin 128 of a coordinate system (e.g., a Cartesian coordinate system) of the housing 110 of the implantable blood pump 100 may be measured during assembly of the pump 100. The origin 128 may be located at a center of the flow conduit 103 or at a center of the stator 120. The origin 128 is provided as input to the instability detection algorithm 900 described in FIG. 9.

At step 902, the instability detection algorithm 900 starts. The instability detection algorithm 900 may proceed with computing a target position 129 for the rotor 140 of the pump 100. In an aspect, the target position of the rotor 140 of the pump 100 may be computed, wherein the target position 129 may be a position within a flow conduit 103 of the pump 100 where power consumption for rotor levitation may be reduced. For example, the target position 129 may be a position within the flow conduit at which a DC component of a levitation current of the rotor 140 is computed to be minimal.

At step 904, the algorithm 900 may proceed with computing of a displacement 132 of the target position 129 from the origin 128 of the housing coordinate system. In a general aspect, the Hall sensor described above may be used to measure or monitor the positions of the rotor. For example the output voltage of the Hall sensor may provide a measure for the displacement 132 of the rotor from the origin 128. The algorithm 900 may receive the output voltage of the Hall sensor. In an aspect, the Hall sensor and the algorithm may be implemented in the implantable blood pump. For example, the displacement 132 may provide information about the gap 108 for a fluid (e.g., blood) to pass the rotor in the flow conduit 103. For example, the gap 108 may be between the inner wall 115 of the pump 100 and the magnet 141 of the rotor 140. For example, it may be derived from the gap 108 if sufficient blood can pass through the gap 108 to fulfill a certain predefined blood flow through the pump 100. For instance, hydrodynamic measures may be computed from the gap 108 and/or the target position 129. This provides information for adjusting one or more functional parameters of the pump 100 to optimize operation of the pump 100.

At step 905, the algorithm 900 may proceed with sending a moving command to the motor of the pump 100 for moving the rotor 140 to the target position 129. For example, a force may be applied to the magnet 141 of the rotor 140 to move the rotor 140 to the target position 129.

At step 906, the algorithm 900 may send a command to the motor for starting the rotor 140.

At step 907, the algorithm 900 may initiate a computing of positional deviations of the rotor 140 from the target position 129 in two radial directions during operation of the rotor. For example, the two radial directions may be orthogonal to each other. For example the two radial directions may lie in a plane that is perpendicular to the rotation axis of the rotor. In a general aspect, the Hall sensor described above may be used to measure or monitor the positions of the rotor. For example the output voltage of the Hall sensor may provide a measure for the positional deviation of the rotor 140 from the target position 129. The algorithm 900 may receive the output voltage of the Hall sensor. In an aspect, the Hall sensor and the algorithm 900 may be implemented at the implantable blood pump. In an aspect, the algorithm is implemented at the blood pump, in an internal controller off the pump, an external controller, or a combination thereof.

At step 908, the algorithm 900 may compute positional noise 133 values of the rotor 140 from the deviations. For example, the noise value may be a RMS noise. For example, the positional noise 133 value may be a moving average of an absolute magnitude of a displacement of the rotor 140 from the target position 129. For example, a sampling rate for the moving average may be 20 kilohertz. In the exemplary embodiment, the pump includes a 2-pole motor. One will appreciate that the invention may be applied equally to motors with any number of poles. If the motor has more poles (e.g. four), the sampling rate may need to be increased to a higher frequency.

At step 909, the algorithm may output the displacement and/or the positional noise 133 values to a control unit that is located external to a patient that has the blood pump 100 implanted in her or his body.

At step 910, the instability detection algorithm 900 ends.

The particular aspects or implementations of the optimized start-up algorithm described in FIGS. 7 to 9 may provide one or more of the following advantages. First, positional measures of a rotor in an implantable blood pump may be monitored during operation of the pump. For example, impeller displacements or noise of the rotor position during operation of the pump may be sensed and provided to a control unit that may be external to the body of a patient carrying the pump. A stable rotation of the rotor may reduce the current consumption of the pump, e.g. by reducing the number of required restarts of the rotor and consequently the number of required take-offs. This may circumvent high current consumption and high peak currents during the operation of the rotor. The consequences can be worse in cases of extreme instability. For example, in extreme cases the pump can fail. Instability can also lead to the rotor contacting the housing or stator, which in turn can create debris in the blood circuit and present associated risks. Second, a target position of a rotor of the implantable blood pump may be computed, wherein the target position is a position within a flow conduit of the pump where power consumption for rotor levitation may be reduced. For example, the target position may be a position within the flow conduit at which a DC component of a levitation current of the rotor is computed to be minimal. Third, a gap for a fluid (e.g., blood) to pass the rotor in the fluid conduit may be computed from the target position. For example, the gap may be between an inner wall of the pump and a magnet of the rotor. For example, it may be derived from the gap if sufficient blood can pass through the gap to fulfill a certain predefined blood flow through the pump. For instance, hydrodynamic measures may be computed from the gap and/or the target position. This provides information for adjusting one or more functional parameters of the pump to optimize operation of the pump. Fourth, the computing of the target position may provide information about a magnetic field distributed within the pump. For example, a strength and/or field lines of a magnetic field inside the pump may be computed from the target position. This provides data for adjusting one or more functional parameters of the pump to optimize operation of the pump. Fifth, a compact, lightweight power supply may be sufficient to provide the current required for the instability detection algorithm. Sixth, larger deviations for the production of the components in the pump may be tolerated. For example, the tolerances regarding eccentricity may be increased by the instability detection algorithm.

A number of aspects or implementations have been described. Nevertheless, it will be understood that various modifications may be made without departing from the scope of the claimed invention. For example, the cap 118 can be engaged with the peripheral wall 116 using a different attachment mechanism or technique, including snap-fit engagement, adhesives, or welding. Additionally, while the cap 118 has been described as defining the outlet opening 105 and the chamfered edge 114, the outlet opening 105 and/or the chamfered edge 114 can be defined by the peripheral wall 116 or by both the peripheral wall 116 and the cap 118. Similarly, the dividing wall 115 can be formed as part of the cap 118.

Additionally, the rotor 140 can include two or more permanent magnets. The number and configuration of the pole pieces 123 can also be varied. The operation of the control electronics 130 is selected to account for the number and position of pole pieces of the stator and permanent magnets of the rotor. Also, the cap 118 can be engaged with the peripheral wall using other techniques, such as adhesives, welding, snap-fit, shrink-fit, or other technique or structure. Similarly, the first face 111 may be formed from a separate piece of material than the peripheral wall 116 and the first face 111, including the inlet cannula 112, can be attached to the peripheral wall 116, such as by welding, after the control electronics 130 and the stator 120 have been mounted in the internal compartment 117. The shroud 145 may be omitted and optionally replaced by other flow control devices to achieve a desired pump efficiency. As another option, the control electronics 130 can be located external to the pump 100, such as in a separate housing implanted in the patient's abdomen, or external to the patient's body.

In some implementations, the dimensions of the housing 110 can be larger or smaller than those described above. Similarly, the ratio of the width W of the housing 110 to the thickness T of the housing can be different than the ratio described above. For example, the width W can be from about 1.1 to about 5 times greater than the thickness T. Additionally, the permanent magnet 141 of the rotor 140 can include two or more pairs of north and south magnetic poles. While the peripheral wall 116 and the dividing wall 115 are illustrated as cylinders having circular cross-sectional shapes, one or both can alternatively be formed having other cross-sectional shapes, such as oval, or an irregular shape. Similarly, the peripheral wall 116 can be tapered such that the housing does not have a constant width W from the first face 111 to the second face 113.

As mentioned above, in some implementations, the blood pump 100 can be used to assist a patient's heart during a transition period, such as during a recovery from illness and/or surgery or other treatment. In other implementations, the blood pump 100 can be used to partially or completely replace the function of the patient's heart on a generally permanent basis, such as where the patient's aortic valve is surgically sealed. In a particular aspect described herein, the Hall sensor may allow to monitor the position of the rotor of the pump 100 and may thereby help to ensure a proper operating status of the implantable blood pump 100.

As used in the present disclosure, the term "computer" ort "controller" is intended to encompass any suitable processing device.

Regardless of the particular implementation, "procedure" or "algorithm" may include computer-readable instructions, firmware, wired or programmed hardware, or any combination thereof on a tangible and non-transitory medium operable when executed to perform at least the processes and operations described herein. Indeed, each procedure or algorithm component may be fully or partially written or described in any appropriate computer language including C, C++, Java, Visual Basic, assembler, Perl, any suitable version of 4GL, as well as others.

The figures and accompanying description illustrate example processes and computer-implementable techniques. It will be understood that the processes are for illustration purposes only and that the described or similar techniques may be performed at any appropriate time, including concurrently, individually, or in combination. In addition, many of the steps in these processes may take place simultaneously, concurrently, and/or in different orders or combinations than shown.

Thus, particular aspects of the subject-matter have been described. Other aspects or embodiments are within the scope of the following claims. In some cases, the actions recited in the claims can be performed in a different order or combination and still achieve desirable results. In certain aspects, multitasking and parallel processing may be advantageous.

In other words, although this disclosure has been described in terms of certain implementations and aspects, generally associated methods, alterations and permutations of these implementations and methods will be apparent to those skilled in the art. Accordingly, the above description of example implementations does not define or constrain this disclosure. Other changes, substitutions and alterations are also possible without departing from the scope of this disclosure.

Aspects of the subject-matter and the operations described in this specification can be implemented in digital electronic circuitry, or in computer software, firmware, or hardware, including the structures disclosed in this specification and their structural equivalents, or in combinations of one or more of them. Embodiments of the subject-matter described in this specification can be implemented as one or more computer programs, i.e., one or more modules of computer program instructions, encoded on computer storage medium for execution by, or to control the operation of a data processing apparatus. Alternatively or in addition, the program instructions can be encoded on an artificially-generated propagated signal, e.g., a machine-generated electrical, optical, or electromagnetic signal, that is generated to encode information for transmission to suitable receiver apparatus for execution by a data processing apparatus. A computer storage medium can be, or be included in, a computer-readable storage device, a computer-readable storage substrate, a random or serial access memory array or device, or a combination of one or more of them. Moreover, while a computer storage medium is not a propagated signal, a computer storage medium can be a source or destination of computer program instructions encoded in an artificially-generated propagated signal. The computer storage medium can also be, or be included in, one or more separate physical components or media (e.g., multiple CDs, disks, or other storage devices).

The operations described in this specification can be implemented as operations performed by a data processing apparatus on data stored on one or more computer-readable storage devices or received from other sources.

The term "computer", "controller", "server", "processor" or "processing device" encompasses all kinds of apparatus, devices, and machines for processing data, including by way of example a programmable processor, a computer, a system on a chip, or multiple ones, or combinations of the foregoing. The apparatus can include special purpose logic circuitry, e.g., an FPGA (field programmable gate array) or an ASIC (application-specific integrated circuit). The apparatus can also include, in addition to hardware, code that creates an execution environment for the computer program in question, e.g., code that constitutes processor firmware, a protocol stack, a database management system, an operating system, a cross-platform runtime environment, a virtual machine, or a combination of one or more of them. The apparatus and operating environment can realize various different computing model infrastructures.

A computer algorithm (also known as a program, software, software application, script, or code) can be written in any form of programming language, including compiled or interpreted languages, declarative or procedural languages, and it can be deployed in any form, including as a stand-alone program or as a module, component, subroutine, object, or other unit suitable for use in a computing environment. A computer program may, but need not, correspond to a file in a file system. A program can be stored in a portion of a file that holds other programs or data (e.g., one or more scripts stored in a markup language document), in a single file dedicated to the program in question, or in multiple coordinated files (e.g., files that store one or more modules, sub-programs, or portions of code). A computer program can be deployed to be executed on one computer or on multiple computers that are located at one site or distributed across multiple sites and interconnected by a communication network.

The processes and logic flows described in this specification can be performed by one or more programmable processors executing one or more computer programs to perform actions by operating on input data and generating output. The processes and logic flows can also be performed by, and apparatus can also be implemented as, special purpose logic circuitry, e.g., an FPGA (field programmable gate array) or an ASIC (application-specific integrated circuit).

Processors suitable for the execution of a computer program include, by way of example, both general and special purpose microprocessors, and any one or more processors of any kind of digital computer. Generally, a processor will receive instructions and data from a read-only memory or a random access memory or both. The essential elements of a computer may be a processor for performing actions in accordance with instructions and one or more memory devices for storing instructions and data. Generally, a computer or computing device 200 will also include, or be operatively coupled to receive data from or transfer data to, or both, one or more mass storage devices for storing data, e.g., magnetic, magneto-optical disks, or optical disks. However, a computer or computing device need not have such devices. Moreover, a computer or computing device can be embedded in another device, e.g., a mobile telephone, a personal digital assistant (PDA), a mobile audio or video player, a game console, a Global Positioning System (GPS) receiver, or a portable storage device (e.g., a universal serial bus (USB) flash drive), to name just a few. Devices suitable for storing computer program instructions and data include all forms of non-volatile memory, media and memory devices, including by way of example semiconductor memory devices, e.g., EPROM, EEPROM, and flash memory devices; magnetic disks, e.g., internal hard disks or removable disks; magneto-optical disks; and CD-ROM and DVD-ROM disks. The processor and the memory can be supplemented by, or incorporated in, special purpose logic circuitry.

To provide for interaction with a user, embodiments of the subject-matter described in this specification can be implemented on a computer having a non-flexible or flexible screen 201, e.g., a CRT (cathode ray tube), LCD (liquid crystal display) or OLED (organic light emitting diode) monitor, for displaying information to the user and a keyboard and a pointer 205, e.g., a finger, a stylus, a mouse or a trackball, by which the user can provide input to the computer. Other kinds of devices can be used to provide for interaction with a user as well; for example, feedback provided to the user can be any form of sensory feedback, e.g., touch feedback, visual feedback, auditory feedback, or tactile feedback; and input from the user can be received in any form, including acoustic, speech, touch or tactile input. In addition, a computer can interact with a user by sending documents to and receiving documents from a device that is used by the user; for example, by sending web pages to a web browser on a user's user device in response to requests received from the web browser.

Embodiments of the subject-matter described in this specification can be implemented in a computing system that includes a back-end component, e.g., as a server 300, or that includes a middleware component, e.g., an application server, or that includes a front-end component, e.g., a user computer having a graphical user interface or a Web browser through which a user can interact with an implementation of the subject-matter described in this specification, or any combination of one or more such back-end, middleware, or front-end components. The components of the system can be interconnected by any form or medium of digital data communication, e.g., a communication network. Examples of communication networks include a local area network ("LAN") and a wide area network ("WAN"), an inter-network (e.g., the Internet), and peer-to-peer networks (e.g., ad hoc peer-to-peer networks).

The computing system can include users and servers. A user and server are generally remote from each other and typically interact through a communication network. The relationship of user and server arises by virtue of computer programs running on the respective computers and having a user-server relationship to each other. In some embodiments, a server transmits data (e.g., an HTML page) to a user device (e.g., for purposes of displaying data to and receiving user input from a user interacting with the user device). Data generated at the user device (e.g., a result of the user interaction) can be received from the user device at the server.

Certain features that are described in this specification in the context of separate embodiments can also be implemented in combination in a single embodiment. Conversely, various features that are described in the context of a single embodiment can also be implemented in multiple embodiments separately or in any suitable sub-combination.

In general, the separation of various system components in the aspects described above should not be understood as requiring such separation in all implementations, and it should be understood that the described program components and system components can generally be integrated together in a single software or hardware product or packaged into multiple software or hardware products.

The preceding figures and accompanying description illustrate example processes and example devices. But example components contemplate using, implementing, or executing any suitable technique for performing these and other tasks. It will be understood that these processes and parts are for illustration purposes only and that the described or similar techniques may be performed at any appropriate time, including concurrently, individually, in parallel, and/or in combination. In addition, many of the steps or parts in these processes may take place simultaneously, concurrently, in parallel, and/or in different orders than as shown. Moreover, components with additional parts, fewer parts, and/or different parts, may be used so long as the devices remain appropriate.

In other words, although this disclosure has been described in terms of certain aspects, implementations, examples or generally associated methods, alterations and permutations of these aspects, implementations or methods will be apparent to those skilled in the art. Accordingly, the above description of example aspects, implementations or embodiments do not define or constrain this disclosure. Other changes, substitutions, and alterations are also possible without departing from the scope of this disclosure.

## Claims

1. A blood pump system comprising:
a housing (110);
a rotary motor comprising a stator (120) and a rotor (140), the rotor (140) having permanent magnetic poles for magnetic levitation of the rotor (140);
a controller configured to calculate, during a rotation of the rotor (140), geometric deviations of a current position of the rotor (140) from a predefined target position (129);
**characterized in that** the controller is further configured to calculate positional noise values (133) based on the geometric deviations.

2. The blood pump system of claim 1, wherein the controller is configured to output the geometric deviations or a figure of merit for a stability of the rotor based on the geometric deviations.

3. The blood pump system of claim 2, wherein the figure of merit is a moving average of absolute magnitudes of the geometric deviations, wherein the geometric deviations are calculated with a sampling rate of about 20 kilohertz.

4. The blood pump system of claim 1, wherein the geometric deviations are calculated with a sampling rate of about 20 kilohertz.

5. The blood pump system of claim 1, wherein the housing (110) is an implantable blood pump housing, wherein the rotary motor and the controller are positioned within the implantable blood pump housing.

6. The blood pump system of any one of claims 1 to 5, wherein the geometric deviations are calculated based on an output voltage of a Hall sensor that is included in the housing (110).

7. The blood pump system of claim 1, wherein the controller is configured to:
determine a target position (129) of the rotor (140);
calculate a positional displacement of the target position (129) from a predefined origin of a coordinate system of a housing (110) of the pump; and
calculate, during the rotation of the rotor (140), geometric deviations of the current position of the rotor (140) from the target position (129).

8. The blood pump system of claim 1, wherein the controller is configured to:
control the rotary motor to move the rotor (140) to towards a target starting position; and
when the rotor (140) is located at within a predetermined volume around the target starting position, control the motor to start rotating the rotor (140).

9. The blood pump system of claim 8, wherein the target starting position is a position within the housing (110) where a power consumption for the magnetic levitation is calculated to be below a predefined power threshold.

10. The blood pump system of claim 8, wherein the target starting position is a position within the housing (110) at which a DC component of a levitation current of the rotor (140) is at a minimum.

11. The blood pump system of claim 1, wherein the positional noise values comprise RMS noise.

## Patentansprüche

1. Blutpumpensystem umfassend:
ein Gehäuse (110);
einen Rotationsmotor, der einen Stator (120) und einen Rotor (140) umfasst, wobei der Rotor (140) permanentmagnetische Pole zum magnetischen Schweben des Rotors (140) umfasst;
ein Controller, der ausgelegt ist, während einer Rotation des Rotors (140) geometrische Abweichungen einer momentanen Position des Rotors (140) von einer vorbestimmten Zielposition (129) zu berechnen;
**dadurch gekennzeichnet, dass**
der Controller weiterhin ausgelegt ist Positionsrauschwerte (133) basierend auf den geometrischen Abweichungen zu berechnen.

2. Blutpumpensystem nach Anspruch 1, wobei der Controller ausgelegt ist die geometrischen Abweichungen oder einen Bewertungsfaktor einer Stabilität des Rotors basierend auf den geometrischen Abweichungen auszugeben.

3. Blutpumpensystem nach Anspruch 2, wobei der Bewertungsfaktor ein gleitender Mittelwert der absoluten Magnituden der geometrischen Abweichungen ist, wobei die geometrischen Abweichungen mit einer Abtastfrequenz von ungefähr 20 Kilohertz berechnet werden.

4. Blutpumpensystem nach Anspruch 1, wobei die geometrischen Abweichungen mit einer Abtastfrequenz von ungefähr 20 Kilohertz berechnet werden.

5. Blutpumpensystem nach Anspruch 1, wobei das Gehäuse (110) ein implantierbares Blutpumpengehäuse ist, wobei der Rotationsmotor und der Controller innerhalb des implantierbaren Blutpumpengehäuses positioniert sind.

6. Blutpumpensystem nach irgendeinem der Ansprüche 1 bis 5, wobei die geometrischen Abweichungen basierend auf einer Spannungsausgabe eines Hall-Sensors, der in dem Gehäuse (110) beinhaltet ist, berechnet werden.

7. Blutpumpensystem nach Anspruch 1, wobei der Controller ausgelegt ist zum:
Bestimmen einer Zielposition (129) des Rotors (140);
Berechnen einer positionellen Dislokation der Zielposition (129) von einem vorbestimmten Ursprung eines Koordinatensystems eines Gehäuses (110) der Pumpe; und
Berechnen von geometrischen Abweichungen der momentanen Position des Rotors (140) von der Zielposition (129) während der Rotation des Rotors (140).

8. Blutpumpensystem nach Anspruch 1, wobei der Controller ausgelegt ist zum:
Steuern des Rotationsmotors, um den Rotor (140) in Richtung einer Ziel-Startposition zu bewegen; und
Steuern des Motors, um Rotieren des Rotors (140) zu starten, wenn sich der Rotor (140) innerhalb eines vorbestimmten Volumens um die Ziel-Startposition herum befindet.

9. Blutpumpensystem nach Anspruch 8, wobei die Ziel-Startposition eine Position innerhalb des Gehäuses (110) ist, für die ein unterhalb eines vorbestimmten Leistungsgrenzwerts bleibender Leistungsverbrauch für das magnetische Schweben berechnet ist.

10. Blutpumpensystem nach Anspruch 8, wobei die Ziel-Startposition eine Position innerhalb des Gehäuses (110) ist, an der sich ein Gleichstromanteil eines Schwebestroms des Rotors (140) an einem Minimum befindet.

11. Blutpumpensystem nach Anspruch 1, wobei die Positionsrauschwerte RMS-Rauschen umfassen.

## Revendications

1. Système de pompe à sang comprenant :
un boîtier (110) ;
un moteur rotatif comprenant un stator (120) et un rotor (140), le rotor (140) ayant des pôles magnétiques permanents pour la lévitation magnétique du rotor (140) ;
un dispositif de commande configuré pour calculer, pendant une rotation du rotor (140), des écarts géométriques d'une position actuelle du rotor (140) à partir d'une position cible prédéfinie (129) ;
**caractérisé en ce que** le dispositif de commande est en outre configuré pour calculer des valeurs de bruit de position (133) sur la base des écarts géométriques.

2. Système de pompe à sang selon la revendication 1, le dispositif de commande étant configuré pour délivrer les écarts géométriques ou un facteur de mérite pour une stabilité du rotor sur la base des écarts géométriques.

3. Système de pompe à sang selon la revendication 2, le facteur de mérite étant une moyenne mobile des amplitudes absolues des écarts géométriques, les écarts géométriques étant calculés avec une fréquence d'échantillonnage d'environ 20 kilohertz.

4. Système de pompe à sang selon la revendication 1, les écarts géométriques étant calculés avec un taux d'échantillonnage d'environ 20 kilohertz.

5. Système de pompe à sang selon la revendication 1, le boîtier (110) étant un boîtier de pompe à sang implantable, le moteur rotatif et le dispositif de commande étant positionnés à l'intérieur du boîtier de pompe à sang implantable.

6. Système de pompe à sang selon l'une quelconque des revendications 1 à 5, les écarts géométriques étant calculés sur la base d'une tension de sortie d'un capteur à effet hall qui est inclus dans le boîtier (110).

7. Système de pompe à sang selon la revendication 1, le dispositif de commande étant configuré pour :
déterminer une position cible (129) du rotor (140) ;
calculer un déplacement de position de la position cible (129) à partir d'une origine prédéfinie d'un système de coordonnées d'un boîtier (110) de la pompe ; et
calculer, pendant la rotation du rotor (140), des écarts géométriques de la position actuelle du rotor (140) à partir de la position cible (129).

8. Système de pompe à sang selon la revendication 1, le dispositif de commande étant configuré pour :
commander le moteur rotatif pour déplacer le rotor (140) vers une position de départ cible ; et
lorsque le rotor (140) est situé à l'intérieur d'un volume prédéterminé autour de la position de départ cible, commander le moteur pour démarrer la rotation du rotor (140).

9. Système de pompe à sang selon la revendication 8, la position de départ cible étant une position à l'intérieur du boîtier (110) où une consommation d'énergie pour la lévitation magnétique est calculée pour être inférieure à un seuil d'énergie prédéfini.

10. Système de pompe à sang selon la revendication 8, la position de départ cible étant une position à l'intérieur du boîtier (110) au niveau de laquelle une composante continue d'un courant de lévitation du rotor (140) est à un minimum.

11. Système de pompe à sang selon la revendication 1, les valeurs de bruit de position comprenant un bruit RMS.
